# EUROPÄISCHE PATENTSCHRIFT

(11) **EP 1 568 347 B1**
(45) Veröffentlichungstag und Bekanntmachung des Hinweises auf die Patenterteilung: **13.08.2008**
(21) Anmeldenummer: 05001610.4
(22) Anmeldetag: 27.01.2005
(51) Int. Cl.: A61K 6/083

(54) **Dispersionen nanoskaliger, nicht-agglomerierter Teilchen zum Einsatz in Dentalmaterialien**
Dispersed nanoscale, non-agglomerated particles for use in dental materials
Particules de taille nanométrique dispersées et non-agglomérées à usage de soins dentaires

(30) Priorität: 18.02.2004 DE 102004008206
(43) Veröffentlichungstag der Anmeldung: 31.08.2005
(73) Patentinhaber: Heraeus Kulzer GmbH, 63450 Hanau (DE)
(72) Erfinder: Ruppert, Klaus Dr., 63477 Maintal (DE); Erdrich, Albert Dr., 61231 Bad Nauheim (DE); Grundler, Andreas Dr., 42117 Wuppertal (DE)
(74) Vertreter: Kühn, Hans-Christian

(56) Entgegenhaltungen:
- EP-A- 0 460 560
- EP-A- 0 803 240
- EP-A- 1 142 917
- EP-A- 1 366 112
- MOSZNER, NORBERT; KLAPDOHR, SIMONE: "Nanotechnology for dental composites" INTERNATIONAL JOURNAL OF NANOTECHNOLOGY, Bd. 1, Nr. 1-2, 2004, Seiten 130-156, XP008049452 Switzerland

## Beschreibung

Die Erfindung betrifft Dentalmaterialien enthaltend Dispersionen nanoskaliger Kieselsäure-Teilchen.

Es wurde bereits vorgeschlagen, nanoskalige, nicht-agglomerierte Kieselsäure-Teilchen mit Teilchengrößen über 10 nm in organischen Dispersionen bei der Herstellung von Dentalmaterialien einzusetzen. Dadurch werden die traditionell verwandten, stark agglomerierten, pyrogenen Kieselsäuren zumindest teilweise ersetzt, und Eigenschaften wie mechanische Festigkeit und Transparenz konnten verbessert werden (EP 803 240 A2, DE 196 17 931 A1, US 5,036,006).

WO03/72659A1 beschreibt ein Verfahren zur Reduktion der durchschnittlichen Partikelgröße von Ta-, Nb-, In- oder Sn-Oxid-Teilchen oder Agglomeraten mittels Magnetfeldern. Die optional getrockneten Teilchen können dann mit Dentalmonomeren vermischt werden (S. 15).

In WO03/55939 wird ein Ultraschallverfahren beschrieben, das zu Dispersionen von anorganischen Partikeln mit Teilchengrößen zwischen 0,1 und 250 nm führt.

WO00/69392 beschreibt oberflächenmodifizierte Nanoteilchen aus Oxiden von Nb, In, Ti, Zn, Zr, Sn, Ce, Hf, Ta, W, Bi Si, vorzugsweise aus ZrO₂ mit Adhäsionspromoter und organofunktionellem Bindemittel mit Teilchengrößen von 10 bis 150 nm, bevorzugt 50-100 nm. Die Teilchen eignen sich für lichthärtende Dentalmaterialien.

WO01/52618 beschreibt das Dispergieren oberflächenstabilisierter kolloidaler Kieselsäure in Polymerlösungen.

EP1366112A1 (WO0283776) beschreibt disperse Phasen enthaltend amorphes SiO₂ mit mittleren Teilchengrößen von 3-50 nm, bevorzugt zwischen 10 und 25 nm, das in Gemischen von nichtradikalisch umsetzbaren Monomeren und Polymeren vorliegt.

Es wurde nun gefunden, dass Dentalmaterialien, hergestellt aus polymerisierbaren Dentalmaterialien, die ihrerseits aus Nanoteilchen aus SiO₂ oder dessen Gemischen oder Mischoxiden mit ZrO₂ und/oder Al₂O₃ und/oder TiO₂ , die durchschnittliche Teilchengrößen von 1 bis 10 nm, besonders 1-8 nm und ganz besonders 2-6 nm aufweisen und aus einem Monomer- oder Monomergemisch-Sol hergestellt sind, überraschend günstige Eigenschaften in den Dentalmaterialien bewirken.

Hierbei können die Nanoteilchen bevorzugt allein oder aber auch in Kombination mit einem geringen Anteil an herkömmlichen, agglomerierten Füllstoffen (wie z.B. Fällungs- oder pyrogenen Kieselsäuren) oder Dentalgläsern eingesetzt werden.

Die Erfindung betrifft somit polymerisierbare Dentalmaterialien enthaltend ein Sol aus einem Monomer oder einer Monomermischung mit darin dispergierten Nanoteilchen, die eine durchschnittliche Teilchengröße von 1 bis <10 nm aufweisen und überwiegend nicht-agglomeriert sind, wobei die Nanoteilchen aus SiO₂ oder dessen Gemischen oder Mischoxiden mit ZrO₂ und/oder Al₂O₃ und /oder TiO₂ bestehen.. Bevorzugt handelt es sich um monodisperse Teilchen.

Unter einem Sol oder Kolloid versteht man eine Suspension, in der die disperse Phase so kleine Durchmesser aufweist (ca. 1 -1000 nm), dass auf die Partikel überwiegend van-der Waals-Kräfte sowie elektrostatische Kräfte wirken und die Gravitationskräfte vernachlässigbar sind.

Die Nanoteilchen liegen in der Regel oberflächenmodifiziert vor. Dazu werden beispielsweise bekannte Silanisierungsmittel wie Silan A-174 (gamma-Methacryloxypropyl-trimethoxysilan, Dow Corning Corp.) verwendet. Es sind aber auch andere Arten der Oberflächenmodifizierung möglich. Zur Herstellung der erfindungsgemäßen Dentalmaterialien vermischt man die Dispersionen zweckmäßig mit den weiteren Komponenten, wie weitere, herkömmliche Füllstoffe, Initiatoren, Stabilisatoren oder Pigmente.

Die erfindungsgemäßen Dentalmaterialien weisen folgende Vorteile auf:
- sehr hohe Transparenz; Transparenz eines nicht eingefärbten Komposits unter Verwendung von Nanofüllern (Teilchengröße ca. 5 nm): 86,75 %; bei Verwendung von Nanofüllern (Teilchengröße ca. 20 nm): 77,29 %
- deutliche Verbesserung der mechanischen Eigenschaften gegenüber keine oder größere Nanoteilchen enthaltenden Materialien;
- niedrige Viskosität, was dazu führt, dass höhere Füllstoffgehalte möglich sind.

Als Monomere kommen die auf dem Dentalgebiet üblichen Monomere in Betracht: Beispiele sind radikalisch polymerisierbare monofunktionelle Monomere wie Mono(meth)acrylate, Methyl-, Ethyl-, Butyl-, Benzyl-, Furfuryl- oder Phenyl(meth)acrylat, polyfunktionelle Monomere wie polyfunktionelle Acrylate bzw. Methacrylate, z.B. Bisphenol-A-di(meth)acrylat, Bis-GMA (ein Additionsprodukt aus Methacrylsäure und Bisphenol-A-diglycidylether), UDMA ("Urethandimethacrylat", z.B. ein Additionsprodukt aus 2-Hydroxyethylmethacrylat und 2,2,4-Hexamethylendiisocyanat), Di-, Tri- oder Tetraethylenglykoldi(meth)acrylat, Decandiol-di(meth)acrylat, Dodecandioldi(meth)acrylat, Hexyldecandioldi(meth)acrylat, Trimethylolpropantri(meth)acrylat, Pentaerythrittetra(meth)acrylat sowie Butandioldi(meth)acrylat.

Bevorzugt sind Bis-GMA, TEDMA (Triethylenglykoldimethacrylat), UDMA (Urethandimethacrylat), TCD-di-HEMA (Bis (methacryloyloxymethyl)tricyclo[5.2.1.0^{2,6}]decan) und TCD-di-HEA (Bis-(acryloyloxymethyl)tricyclo[5.2.1.0^{2,6}]decan)

Als herkömmliche Füllstoffe kommen neben den bereits erwähnten Oxiden (TiO₂, ZrO₂, Al₂O₃, SiO₂) weitere Metalloxide wie Zinnoxid, Metallsulfate, weitere Oxide der Nebengruppen des Periodensystems, fluoridabgebende Substanzen, pyrogene oder Fällungskieselsäuren, Dentalgläser wie Aluminosilicatgläser oder Fluoroaluminosilicatgläser, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis (SiO₂, ZrO₂ und/oder TiO₂), Metalloxide mit Primärteilchengröße von ca. 40 bis 300 nm, Splitterpolymerisate mit 10-100 µm Teilchengröße (vgl. R. Janda, Kunststoffverbundsysteme, VCH Verlagsgesellschaft, Weinheim, 1990, Seite 225 ff.) oder deren Mischungen in Frage. Zudem können Verstärkungsmittel wie Glasfasern, Polyamid- oder Kohlenstofffasern eingearbeitet werden.

Der Füllstoffgehalt hängt stark von der beabsichtigten Verwendung ab. Zemente enthalten bevorzugt 5 bis 60 Gew.%, besonders 20 bis 60 Gew.%, Veneers oder Seiten- bzw. Frontzahnmaterialien bevorzugt 50 bis 90 Gew.%, insbesondere 60 bis 80 Gew.%, Füllungskomposite bevorzugt 50 bis 90 Gew.%, insbesondere 75 bis 85 Gew.% Füller, jeweils relativ zur Gesamtmasse des Dentalmaterials.

Des weiteren können die erfindungsgemäßen Dentalmaterialien weitere, bei Dentalmaterialien übliche Stoffe enthalten, z.B. aus den Gruppen der Pigmente, Stabilisatoren, der antimikrobiellen Additive, UV-Absorber, Thixotropiermittel, Katalysatoren, Photoinitiatoren und Vernetzer.

Solche Additive werden in eher geringen Mengen eingesetzt, insgesamt 0.01 bis 3.0, besonders 0.01 bis 1.0 Gew.% bezogen auf die Gesamtmasse des Dentalmaterials.

Die Aushärtung der Zusammensetzungen kann je nach Art des verwendeten Polymerisationsinitiators durch thermische, photochemische oder redoxinduzierte radikalische Polymerisation erfolgen.

Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie Azobisisobutyroethylester, Azobisisobutyronitril, Azobis-(2-methylpropionamidin)dihydrochlorid, Benzpinakol oder 2,2-Dimethylbenzpinakol.

Bevorzugte Photoinitiatoren sind Benzophenon, Benzoin sowie deren Derivate oder alpha - Diketone oder deren Derivate wie 9,10-Phenanthrenchinon, Diacetyl oder 4,4-Dichlorbenzil. Besonders bevorzugt werden Campherchinon und 2,2-Dimethoxy-2-phenylacetophenon und besonders bevorzugt alpha -Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.
Als Initiatoren für die bei Raumtemperatur durchgeführte Polymerisation werden vorzugsweise Redox-Initiatorkombinationen, wie z.B. Kombinationen von Benzoyl- oder Laurylperoxid mit N,N-Dimethyl-sym.-xylidin oder N,N-Dimethyl-p-toluidin, verwendet.

Die erfindungsgemäßen Dispersionen können z.B. in allen Arten von Füllungskompositen, Veneers, Dentallacken, Fissurenversieglern, Befestigungszementen, Prothesenbasismaterialien, Kronen- und Brückenverblendkompositen, Adhäsiven oder Materialien für künstliche Zähne eingesetzt werden. Entsprechend betrifft die Erfindung auch Dentalmaterialien aus der Gruppe der Füllungskomposite, Befestigungszemente, Prothesenbasismaterialien, Kronen- und Brückenverblendkomposite, Dentallacke, künstliche Zähne und Adhäsive, welche Dispersionen von Nanoteilchen enthalten, die eine durchschnittliche Teilchengröße von 1 bis 10 nm aufweisen und nicht agglomeriert sind.

Die in den erfindungsgemäßen Dentalmaterialien eingesetzten Nanoteilchen-Dispersionen sind nach bekannten Verfahren hergestellt. Z.B. können Sie aus käuflichen wässrigen Solen von amorphem Siliciumdioxid (Hersteller z.B. Nissan Chemicals, Bayer etc.) dadurch erhalten werden, dass zunächst die Oberfläche der SiO₂-Partikel durch Umsetzung, z.B. mit 3-(Meth)acryloyloxypropyltrialkoxysilan, modifiziert wird, anschließend das Wasser als Azeotrop mit einem flüchtigen Alkohol, z.B. Isopropanol, abdestilliert wird und die alkoholische Dispersion in das gewünschte Monomer oder die gewünschte Monomermischung, z.B. 2-Hydroxyethylmethacrylat oder Triethylenglycoldimethacrylat, gegeben wird. Entfernen des Alkohols im Vakuum führt dann zum gewünschten Monomer-Sol. Ähnliche Monomer-Sole, allerdings mit größerem Teilchendurchmesser (ca. 20 nm), werden kommerziell unter der Bezeichnung Nanocrylvon der Firma hanse chemie AG sowie HighLink OG von der Firma Clariant angeboten. Solche wurden für das Beispiel 2 eingesetzt.

Die Erfindung betrifft somit auch ein Verfahren zur Herstellung von Nanoteilchen-Dispersionen in einem Monomer oder einer Monomermischung mit folgenden Schritten
A Bereitstellung von Nanoteilchen aus SiO₂ und /oder ZrO₂ und/oder Al₂O₃ und /oder TiO₂ in einem wässrigen Sol
B Behandeln mit einem Oberflächenmodifizierer
C Einarbeiten des wässrigen Sols in einen flüchtigen Alkohol wie z.B. Isopropanol
D Entfernen des Wassers durch azeotrope Destillation
E Einarbeiten des alkoholischen Sols in ein Monomer oder eine Monomermischung
F Entfernen des Alkohols aus dem Monomer/der Monomermischung, z.B. durch Destillation

Die Erfindung betrifft auch ein Verfahren zur Herstellung eines polymerisierbaren Dentalmaterials, wobei eine wie oben beschrieben hergestellte Nanoteilchendispersion mit Füllstoffen und Initiatoren sowie gegebenenfalls weiteren üblichen Additiven
vermischt wird.

Die Erfindung wird anhand der folgenden Beispiele näher erläutert, ohne sie darauf zu beschränken:

### Beispiel 1:

In eine Monomermischung, bestehend aus den Komponenten

| | |
|---|---|
| Bis-GMA | 14,0 Gew.% |
| TEDMA | 6,0 Gew.% |

werden nanoskalige, nicht-agglomerierte SiO₂-Teilchen mit einer durchschnittlichen Teilchengröße von 4 nm durch Zugabe einesentsprechenden alkoholischen Sols eindispergiert. Anschließend wird der Alkohol im Vakuum entfernt. Der Restalkoholgehalt und Restwassergehalt des Monomergemisch-Sols liegt bei jeweils < 1 Gew. %. Die Konzentration an SiO₂ wird so gewählt, dass im polymerisierbaren Dentalmaterial nach Zugabe aller weiteren Komponenten eine Konzentration von 10 Gew. % SiO₂ vorliegt.

Die Acrylatdispersion wird anschließend mit 0,01 Gew.% Stabilisator BHT (4-Methyl-2,6-di-tert-butyl-phenol, CAS 128-37-0) sowie den Initiatoren Campherchinon (0,3 Gew.%) und 0,2 Gew.% 2-Ethylhexyl-4-dimethylaminobenzoat (Quantacure™EHA, CAS 21245-02-3, Great Lakes Chemicals) versetzt.

Zugabe eines silanisierten Dentalglases (Ba-Al-Silikat; 70,0 Gew. %) und innige Vermischung ergeben ein als Füllungskomposit geeignetes Material.

### Beispiel 2: Resultat von Transparenzmessungen

Die Transparenz eines wie in Beispiel 1 beschriebenen, nicht eingefärbten Komposits unter Verwendung von Nanofüllern (Teilchengröße ca. 5 nm) betrug 86,75 %. Bei entsprechender Verwendung von Nanofüllern mit Teilchengröße von ca. 20 nm betrug sie 77,29 %.

## Patentansprüche

1. Polymerisierbares Dentalmaterial enthaltend ein Sol aus einem Monomer oder ein Sol aus einer Monomermischung mit darin dispergierten Nanoteilchen, die eine durchschnittliche Teilchengröße von 1 bis <10 nm aufweisen und überwiegend nicht agglomeriert sind, wobei die Nanoteilchen aus SiO₂ oder dessen Gemischen oder Mischoxiden mit ZrO₂ und/oder Al₂O₃ und/oder TiO₂ bestehen.

2. Polymerisierbares Dentalmaterial nach Anspruch 1, enthaltend zusätzlich mindestens einen weiteren Füllstoff aus der Gruppe der Dentalgläser.

3. Polymerisierbares Dentalmaterial nach einem der vorstehenden Ansprüche, enthaltend zusätzlich agglomerierte Kieselsäuren.

4. Polymerisierbares Dentalmaterial nach einem der vorstehenden Ansprüche, wobei mindestens ein Monomer ein mono- oder polyfunktionelles Acrylat oder Methacrylat ist oder die Monomermischung mindestens ein mono- oder polyfunktionelles Acrylat oder Methacrylat enthält.

5. Polymerisierbares Dentalmaterial nach einem der vorstehenden Ansprüche, wobei die verschiedenen Oxid-Teilchen in Form von deren Mischoxiden vorliegen.

6. Polymerisierbares Dentalmaterial nach einem der vorstehenden Ansprüche, wobei die verschiedenen Oxid-Teilchen in Form von physikalischen Mischungen vorliegen.

7. Polymerisierbares Dentalmaterial nach einem der vorstehenden Ansprüche, wobei die Nanoteilchen eine Oberflächenmodifizierung aufweisen.

8. Polymerisierbares Dentalmaterial nach einem der vorstehenden Ansprüche, wobei der Oberflächenmodifizierer kovalent auf der Nanoteilchenoberfläche verankert ist.

9. Polymerisierbares Dentalmaterial nach einem der vorstehenden Ansprüche, wobei der Oberflächenmodifizierer kovalent in die Harzmatrix einpolymerisiert.

10. Polymerisierbares Dentalmaterial nach einem der vorstehenden Ansprüche, wobei die Nanoteilchen mono-dispers vorliegen.

11. Polymerisierbares Dentalmaterial nach einem der vorstehenden Ansprüche, wobei die Aushärtung mittels sichtbarem Licht in einem Wellenlängenbereich zwischen 350 und 500 nm erfolgt.

12. Polymerisierbares Dentalmaterial nach einem der vorstehenden Ansprüche aus der Gruppe der Füllungskomposite, Befestigungszemente, Prothesenbasismaterialien, Dentallacke, Materialien für künstliche Zähne, Kronen- und Brückenverblendkomposite und Adhäsive.

13. Polymerisierbares Dentalmaterial nach einem der vorstehenden Ansprüche enthaltend
mindestens ein Monomer der Gruppe Bis-GMA, TEDMA (Triethylenglykoldimethacrylat), UDMA (Urethandimethacrylat, TCD-di-HEMA (Bis (methacryloyloxymethyl)tricyclo[5.2.1.0^{2.6}]decan), TCD-di-HEA (Bis-(acryloyloxymethyl)tricyclo[5.2.1.0^{2,6}]decan) ;
eine Füllermischung aus den Gruppen
- SiO₂ oder dessen Gemischen oder Mischoxiden mit ZrO₂ und/oder Al₂O₃ und /oder TiO₂;
- SiO₂ oder dessen Gemischen oder Mischoxiden mit ZrO₂ und/oder Al₂O₃ und /oder TiO₂ und Dentalgläser.

14. Verfahren zur Herstellung von polymerisierbarem Dentalmaterial nach einem der vorstehenden Ansprüche, **dadurch gekennzeichnet, daß** zunächst eine Nanoteilchen-Dispersion durch
A Bereitstellung eines wäßrigen Sols von Nanoteilchen aus SiO₂ oder dessen Gemischen oder Mischoxiden mit ZrO₂ und/oder Al₂O₃ und /oder TiO₂ oder Gemische aus diesen Oxiden
B Behandeln mit einem Oberflächenmodifizierer
C Einarbeiten des wässrigen Sols in einen flüchtigen Alkohol wie z.B. Isopropanol
D Entfernen des Wassers durch azeotrope Destillation
E Einarbeiten des alkoholischen Sols in ein Monomer oder eine Monomermischung
F Entfernen des Alkohols aus dem Monomer/der Monomermischung
hergestellt wird und diese mit weiteren Füllstoffen und Initiatoren sowie gegebenenfalls weiteren üblichen Additiven vermischt wird.

15. Polymerisierbares Dentalmaterial nach mindestens einem der Ansprüche 1-14, wobei die durchschnittliche Teilchengröße bei 1 bis 8 nm liegt.

16. Polymerisierbares Dentalmaterial nach mindestens einem der Ansprüche 1-14, wobei die durchschnittliche Teilchengröße bei 2 bis 6 nm liegt.

17. Polymerisierbares Dentalmaterial nach mindestens einem der Ansprüche 1-14, wobei die durchschnittliche Teilchengröße bei 4 nm liegt.

## Claims

1. Polymerizable dental material containing a sol comprising a monomer or a sol comprising a monomer mixture with nanoparticles which are dispersed therein, have a mean particle size of 1 to <10 nm and are predominantly unagglomerated, the nanoparticles consisting of SiO₂ or mixtures thereof or mixed oxides with ZrO₂ and/or Al₂O₃ and/or TiO₂.

2. Polymerizable dental material according to Claim 1, additionally containing at least one further filler from the group consisting of the dental glasses.

3. Polymerizable dental material according to either of the above claims, additionally containing agglomerated silicas.

4. Polymerizable dental material according to any of the above claims, wherein at least one monomer is a mono- or polyfunctional acrylate or methacrylate or the monomer mixture contains at least one mono- or polyfunctional acrylate or methacrylate.

5. Polymerizable dental material according to any of the above claims, wherein the various oxide particles are present in the form of their mixed oxides.

6. Polymerizable dental material according to any of the above claims, wherein the various oxide particles are present in the form of physical mixtures.

7. Polymerizable dental material according to any of the above claims, wherein the nanoparticles have a surface modification.

8. Polymerizable dental material according to any of the above claims, wherein the surface modifier is covalently anchored on the nanoparticle surface.

9. Polymerizable dental material according to any of the above claims, wherein the surface modifier is incorporated in the resin matrix covalently by polymerization.

10. Polymerizable dental material according to any of the above claims, wherein the nanoparticles are present in monodisperse form.

11. Polymerizable dental material according to any of the above claims, wherein the curing is effected by means of visible light in a wavelength range between 350 and 500 nm.

12. Polymerizable dental material according to any of the above claims from the group consisting of the filling composites, fixing cements, prosthesis base materials, dental varnishes, materials for artificial teeth, crown and bridge veneer composites and adhesives.

13. Polymerizable dental material according to any of the above claims, containing
at least one monomer from the group bis-GMA, TEDMA (triethylene glycol dimethacrylate), UDMA urethane dimethacrylate, TCD-di-HEMA (bis(methacryloyloxymethyl)tricyclo[5.2.1.0^{2,6}]-decane, TCD-di-HEA (bis(acryloyloxymethyl)-tricyclo[5.2.1.0^{2,6}] decane) ;
a filler mixture from the groups
- SiO₂ or mixtures thereof or mixed oxides with ZrO₂ and/or Al₂O₃ and/or TiO₂;
- SiO₂ or mixtures thereof or mixed oxides with ZrO₂ and/or Al₂O₃ and/or TiO₂ and dental glasses.

14. Process for the production of polymerizable dental material according to any of the above claims, **characterized in that** first a nanoparticle dispersion is prepared by
**A** provision of an aqueous sol of nanoparticles of SiO₂ or mixtures thereof or mixed oxides with ZrO₂ and/or Al₂O₃ and/or TiO₂ or mixtures of these oxides
**B** treatment with a surface modifier
**C** incorporation of the aqueous sol into a volatile alcohol, such as, for example, isopropanol
**D** removal of the water by azeotropic distillation
**E** incorporation of the alcoholic sol into a
monomer or a monomer mixture
**F** removal of the alcohol from the monomer /monomer mixture
and said nanoparticle dispersion is mixed with further fillers and initiators and optionally further customary additives.

15. Polymerizable dental material according to at least one of Claims 1 - 14, wherein the average particle size is 1 to 8 nm.

16. Polymerizable dental material according to at least one of Claims 1 - 14, wherein the average particle size is 2 to 6 nm.

17. Polymerizable dental material according to at least one of Claims 1 - 14, wherein the average particle size is 4 nm.

## Revendications

1. Matériau dentaire polymérisable contenant un sol constitué d'un monomère ou un sol constitué d'un mélange de monomères avec des nanoparticules dispersées dans celui-ci, lesquelles présentent une taille moyenne de particules de 1 à < 10 nm et lesquelles sont de manière prédominante non agglomérées, les nanoparticules étant constituées de SiO₂ ou de mélanges ou d'oxydes mixtes de celui-ci avec ZrO₂ et/ou Al₂O₃ et/ou TiO₂.

2. Matériau dentaire polymérisable selon la revendication 1 contenant de plus au moins une autre charge du groupe des verres dentaires.

3. Matériau dentaire polymérisable selon l'une quelconque des revendications précédentes contenant de plus des acides siliciques agglomérés.

4. Matériau dentaire polymérisable selon l'une quelconque des revendications précédentes, dans lequel au moins un monomère est un acrylate ou un méthacrylate mono- ou polyfonctionnel ou le mélange de monomères contient au moins un acrylate ou méthacrylate mono- ou polyfonctionnel.

5. Matériau dentaire polymérisable selon l'une quelconque des revendications précédentes, dans lequel les différentes particules d'oxyde sont présentes dans la forme de leurs oxydes mixtes.

6. Matériau dentaire polymérisable selon l'une quelconque des revendications précédentes, dans lequel les différentes particules d'oxyde sont présentes dans la forme de mélanges physiques.

7. Matériau dentaire polymérisable selon l'une quelconque des revendications précédentes, dans lequel les nanoparticules présentent une modification de surface.

8. Matériau dentaire polymérisable selon l'une quelconque des revendications précédentes, dans lequel l'agent de modification de surface est ancré de manière covalente sur la surface des nanoparticules.

9. Matériau dentaire polymérisable selon l'une quelconque des revendications précédentes, dans lequel l'agent de modification de surface est polymérisé de manière covalente dans la matrice de résine.

10. Matériau dentaire polymérisable selon l'une quelconque des revendications précédentes, dans lequel les nanoparticules sont présentes dans une forme monodispersée.

11. Matériau dentaire polymérisable selon l'une quelconque des revendications précédentes, dans lequel le durcissement est réalisé au moyen d'une lumière visible dans un domaine des longueurs d'onde comprises entre 350 et 500 nm.

12. Matériau dentaire polymérisable selon l'une quelconque des revendications précédentes choisi parmi des composites de remplissage, des ciments de consolidation, des matériaux de bases de prothèses, des émaux dentaires, des matériaux pour des dents artificielles, des composites de revêtement de couronnes et de bridges et des adhésifs.

13. Matériau dentaire polymérisable selon l'une quelconque des revendications précédentes contenant
au moins un monomère choisi parmi le Bis-GMA, le TEDMA (diméthacrylate de triéthylèneglycol), l'UDMA (diméthacrylate d'uréthane), le TCD-di-HEMA (bis(méthacryloyloxyméthyl)tricyclo[5.2.1.0^{2,6}]décane), le TCD-di-HEA (bis-(acryloyloxyméthyl)tricyclo[5.2.1.0^{2,6}]décane) ;
un mélange de charges choisies parmi
- SiO₂ ou des mélanges ou des oxydes mixtes de celui-ci avec ZrO₂ et/ou Al₂O₃ et/ou TiO₂ ;
- SiO₂ ou des mélanges ou des oxydes mixtes de celui-ci avec ZrO₂ et/ou Al₂O₃ et/ou TiO₂ et des verres dentaires.

14. Procédé pour la préparation d'un matériau dentaire polymérisable selon l'une quelconque des revendications précédentes, **caractérisé en ce que** l'on prépare tout d'abord une dispersion de nanoparticules par
A préparation d'un sol aqueux de nanoparticules de SiO₂ ou de mélanges ou d'oxydes mixtes de celui-ci avec ZrO₂ et/ou Al₂O₃ et/ou TiO₂ ou de mélanges de ces oxydes
B traitement avec un agent de modification de surface
C incorporation d'un sol aqueux dans un alcool volatil comme par exemple l'isopropanol
D élimination de l'eau par distillation azéotrope
E incorporation du sol alcoolique dans un monomère ou dans un mélange de monomères
F élimination de l'alcool à partir du monomère/mélange de monomères
et on mélange celle-ci avec d'autres charges et initiateurs ainsi qu'éventuellement d'autres additifs classiques.

15. Matériau dentaire polymérisable selon au moins l'une des revendications 1-14, dans lequel la taille moyenne de particules est de 1 à 8 nm.

16. Matériau dentaire polymérisable selon au moins l'une des revendications 1-14, dans lequel la taille moyenne de particules est de 2 à 6 nm.

17. Matériau dentaire polymérisable selon au moins l'une des revendications 1-14, dans lequel la taille moyenne de particules est de 4 nm.
